# EUROPÄISCHE PATENTSCHRIFT

(11) **EP 1 531 724 B1**
(45) Veröffentlichungstag und Bekanntmachung des Hinweises auf die Patenterteilung: **28.06.2006**
(21) Anmeldenummer: 02787834.7
(22) Anmeldetag: 26.11.2002
(51) Int. Cl.: A61B 5/05

(54) **ELEKTRODENANORDNUNG**
ELECTRODE SYSTEM
SYSTEME D'ELECTRODES

(30) Priorität: 21.08.2002 DE 10238310
(43) Veröffentlichungstag der Anmeldung: 25.05.2005
(73) Patentinhaber: Viasys Healthcare GmbH, 97204 Höchberg (DE)
(72) Erfinder: EICHLER, Rüdiger, 97225 Zellingen (DE)
(74) Vertreter: Beier, Ralph
(86) Internationale Anmeldenummer: PCT/EP2002/013327
(87) Internationale Veröffentlichungsnummer: WO 2004/021880

(56) Entgegenhaltungen:
- US-A- 4 026 278
- US-A- 5 313 952
- US-B1- 6 272 365

## Beschreibung

Die Erfindung betrifft eine Elektrodenanordnung, insbesondere für die Elektro-Impedanz-Tomographie, gemäß dem Oberbegriff des Anspruchs 1 sowie ein Verfahren zum Anlegen einer derartigen Elektrodenanordnung an ein Messobjekt gemäß Anspruch 24.

Zur Durchführung der sogenannten Elektro-Impedanz-Tomographie bei einem Patienten müssen zahlreiche Elektroden an dem Brustkorb des Patienten befestigt werden, wobei eine genaue Positionierung der Elektroden und eine dauerhafte Kontaktierung der Haut wichtig ist.

Es sind deshalb Elektrodenanordnungen bekannt, bei denen die Elektroden an einem gürtelförmigen Elektrodenträger befestigt sind, der aus einem elastischen Material besteht und zur Messung um den Brustkorb des Patienten herumgelegt wird. Die Fixierung der Elektroden auf der Haut des Patienten erfolgt hierbei mechanisch oder durch Aufkleben der Elektroden. Ähnliche Elektrodenanordnungen mit einem einteiligen, bandförmigen Elektrodenträger sind beispielsweise auch aus US-A-5 313 952 bekannt.

Nachteilig an dieser bekannten Elektrodenanordnung ist jedoch die Tatsache, dass die einzelnen Elektroden in Umfangsrichtung nicht frei positioniert werden können, da sich die Position der einzelnen Elektroden aufgrund der Elastizität des gürtelförmigen Elektrodenträgers und der Querschnittsform des Brustkorbs einstellt und von der Untersuchungsperson nicht oder kaum verändert werden kann. Insbesondere ist es mit der bekannten Elektrodenanordnung nicht möglich, konstante Abstände oder Winkelabstände zwischen den einzelnen Elektroden einzustellen, was jedoch bei der Elektro-Impedanz-Tomographie wichtig ist.

Weiterhin ist aus US-B1-6 272 365 eine Elektrodenanordnung bekannt, die aus einem Elektrodenträger und einem Spannband besteht, wobei der Elektrodenträger einteilig aufgebaut ist, während das Spannband lediglich dazu dient, die gesamte Elektrodenanordnung am Brustkorb einer Versuchsperson zu fixieren, so dass das Spannband selbst keine Elektroden aufweist.

Ferner ist aus US-A-4 026 278 eine herkömmliche Elektrodenanordnung mit einem gürtelförmigen Elektrodenträger bekannt.

Der Erfindung liegt somit die Aufgabe zugrunde, die vorstehend beschriebene bekannte Elektrodenanordnung mit einem gürtelförmigen Elektrodenträger dahingehend zu verbessern, dass die einzelnen Elektroden an dem Brustkorb in der gewünschten Position angebracht werden können.

Die Aufgabe wird, ausgehend von der vorstehend beschriebenen bekannten Elektrodenanordnung gemäß dem Oberbegriff des Anspruchs 1, durch die kennzeichnenden Merkmale des Anspruchs 1 und hinsichtlich eines entsprechenden Verfahrens zum Anlegen einer derartigen Elektrodenanordnung durch die Merkmale des Anspruchs 24 gelöst.

Die Erfindung umfasst die allgemeine technische Lehre, dass die Elektroden an dem gürtelförmigen Elektrodenträger in Längsrichtung bzw. in Umfangsrichtung des gürtelförmigen Elektrodenträgers positionierbar sind, wobei die Positionierung vorzugsweise innerhalb vorgegebener Grenzen frei und bleibend sein soll.

Die Positionierbarkeit der Elektroden wird vorzugsweise dadurch erreicht, dass der gürtelförmige Elektrodenträger mindestens teilweise aus einem plastisch verformbaren Material besteht. Die Position der einzelnen Elektroden auf dem Thorax ist vorzugsweise gleichmäßig mit vorzugsweise konstanten Winkelabständen verteilt und/oder kann von der Untersuchungsperson verändert werden, indem der gürtelförmige Elektrodenträger zwischen den einzelnen Elektroden gedehnt wird, bis sich der gewünschte Elektrodenabstand einstellt. Aufgrund der Plastizität des Materials des gürtelförmigen Elektrodenträgers bleibt dieser Elektrodenabstand auch dann erhalten, wenn die Untersuchungsperson keine Kräfte mehr auf den Elektrodenträger ausübt, was beispielsweise dann der Fall ist, wenn die erfindungsgemäße Elektrodenanordnung an dem Brustkorb eines Patienten angebracht ist.

Vorzugsweise dehnt sich der Elektrodenträger bei einer Dehnung in Längsrichtung über seine Länge im wesentlichen gleichmäßig aus.'Auf diese Weise lassen sich vorteilhaft gleichmäßige Abstände oder Winkelabstände zwischen den benachbarten Elektroden erreichen, was für die Elektro-Impedanz-Tomographie wichtig ist. Der Elektrodenträger besteht deshalb vorzugsweise aus einem Material mit einem homogenen Dehnungsverhalten.

Bei dem plastisch verformbaren Material des gürtelförmigen Elektrodenträgers kann es sich beispielsweise um einen Kunststoff handeln, jedoch sind grundsätzlich auch andere Materialien möglich. Auch ist es nicht erforderlich, dass der gürtelförmige Elektrodenträger vollständig aus einem plastisch verformbaren Material besteht. Es reicht vielmehr aus, wenn jeweils Dehnungsabschnitte zwischen den einzelnen Elektroden aus einem plastisch verformbaren Material bestehen, um eine Positionierung der Elektroden entsprechend der gewünschten Elektrodenposition auf dem Thorax zu ermöglichen.

Die freie Positionierbarkeit der Elektroden in Längsrichtung des Elektrodenträgers kann auch dadurch erreicht werden, dass der Elektrodenträger zwischen den Elektroden in mehreren Lagen übereinander gefaltet ist. Der Abstand der Elektroden läßt sich dann vergrößern, indem der Elektrodenträger in Längsrichtung auseinander gezogen wird, wobei sich der Elektrodenträger teilweise entfaltet.

In den Faltkanten zwischen den einzelnen Lagen des Elektrodenträgers ist hierbei vorzugsweise ein Schutzelement angeordnet, um eine störende Knickbildung zu vermeiden. Dies ist vorteilhaft, da scharfe Knicke die freie Entfaltung des Elektrodenträgers beeinträchtigen und damit die Positionierbarkeit der Elektroden stören würden. Vorzugsweise weist das Schutzelement einen runden Querschnitt auf und hat einen Außendurchmesser, der größer ist als die Dicke des Elektrodenträgers. Das Schutzelement kann beispielsweise aus Kunststoff, wie Polyethylen (PE), bestehen, jedoch sind auch andere Materialien möglich.

Zur Verhinderung einer ungewollten selbständigen Entfaltung des Elektrodenträgers sind die übereinander gefalteten Lagen des Elektrodenträgers vorzugsweise beidseitig mit einem Klebeband bedeckt, das die einzelnen Lagen in der jeweiligen Position fixiert, sofern der Elektrodenträger nicht in Längsrichtung auseinander gezogen wird. Die einzelnen Lagen des Elektrodenträgers bilden hierbei also ein Paket, das von dem Klebeband zusammengehalten und an einer selbständigen Entfaltung gehindert wird.

In einer bevorzugten Ausführungsform der erfindungsgemäßen Elektrodenanordnung sind zur elektrischen Kontaktierung der Elektroden Anschlussleitungen vorgesehen, wobei die Anschlussleitungen an dem gürtelförmigen Elektrodenträger angebracht und in Längsrichtung des gürtelförmigen Elektrodenträgers dehnbar sind, damit eine Beschädigung der Anschlussleitung bei einer Dehnung des gürtelförmigen Elektrodenträgers in Umfangsrichtungen verhindert wird.

Eine Möglichkeit zur Erreichung der Dehnbarkeit der Anschlussleitungen besteht darin, die Anschlussleitungen mäanderförmig zu führen, so dass eine Dehnung des gürtelförmigen Elektrodenträgers in Längs- bzw. Umfangsrichtung nur zu einer geringfügigen Deformation der Anschlussleitungen führt, wodurch eine Beschädigung der Anschlussleitungen bei einer Dehnung des gürtelförmigen Elektrodenträgers verhindert wird.

Eine andere Möglichkeit hierzu besteht jedoch darin, dass die Anschlussleitungen aus einem elektrisch leitfähigen und trotzdem mechanisch dehnbaren Material bestehen, so dass auf eine mäanderförmige Führung der Anschlussleitungen auf dem gürtelförmigen Elektrodenträger verzichtet werden kann.

In einer Variante der Erfindung weist der Elektrodenträger eine Folie auf, wobei die Anschlußleitungen und/oder die Elektroden auf die Folie aufgedruckt sind, was vorteilhaft eine kostengünstige Massenherstellung ermöglicht. Die Folie dient vorzugsweise als mechanischer Träger und kann beispielsweise aus Mylar bestehen.

Vorzugsweise trägt die Folie auf der dem Messobjekt abgewandten Seite eine Isolierschicht, um die Anschlußleitungen gegenüber der Umgebung elektrisch zu isolieren.

Darüber hinaus kann eine Abschirmungsschicht vorgesehen sein, die vorzugsweise auf der dem Messobjekt abgewandten Seite auf die Isolierschicht aufgebracht ist. Eine derartige Abschirmungsschicht besteht aus einem elektrisch leitfähigen Material und wird vorzugsweise auf Massepotential gelegt, um die Anschlußleitungen der Elektroden gegenüber störenden elektromagnetischen Feldern abzuschirmen.

Die Abschirmung kann sowohl ganzflächig als auch je Einzelelektrode ausgebildet sein und auf der gegenüberliegenden Seite des Elektrodenträgers identisch bzw. geringfügig breiter geführt werden. Die Abschirmung kann dabei sowohl gemeinsam geerdet, als auch je Einzelelektrode aktiv mit dem jeweiligen Einzelsignal zur Gleichtaktunterdrückung getrieben sein.

Die elektrische Kontaktierung von Elektrodenbahnen und Abschirmungsbahnen am Stecker erfolgt vorzugsweise durch einen doppelseitigen Steckverbinder. Dieser doppelseitige Stecker kann durch eine Verstärkung am Elektrodenträger angesteckt und z.B. durch eine Druckverbindung gesichert kontaktiert werden.

Erfindungsgemäß besteht der gürtelförmige Elektrodenträger aus mehreren Teilen, die im montiertem Zustand an dem Messobjekt in Längsrichtung des Elektrodenträgers aneinander angrenzen und jeweils paarweise mechanisch und/oder elektrisch miteinander verbunden werden können. Durch diese Aufteilung des gürtelförmigen Elektrodenträgers in mehrere Teile wird die Anbringung der erfindungsgemäßen Elektrodenanordnung insbesondere bei bettlägerigen Patienten erleichtert, bei denen jeweils nur ein Teil des Brustkorbs frei zugänglich ist.

Die mechanische Verbindung der aneinander angrenzenden Teile des gürtelförmigen Elektrodenträgers kann beispielsweise durch eine Steckverbindung oder eine Crimpverbindung erfolgen.

Vorzugsweise werden die einzelnen Teile des gürtelförmigen Elektrodenträgers jedoch getrennt voneinander an dem Messobjekt befestigt, indem die Teile beispielsweise aufgeklebt werden.

Ferner ist in einer vorteilhaften Variante der Erfindung vorgesehen, dass zur elektrischen Kontaktierung des Messobjekts durch die Elektroden eine Kontaktflüssigkeit vorgesehen ist, die in einem Vorratsbehälter bevorratet wird, wobei der Vorratsbehälter an dem gürtelförmigen Elektrodenträger angebracht ist. Hierbei ist vorzugsweise eine optische und/oder akustische Füllstandsanzeige für den Vorratsbehälter vorgesehen, die den Patienten oder die Untersuchungsperson rechtzeitig darüber informiert, wenn die Kontaktflüssigkeit aufgebraucht wird. Die Füllstandsanzeige erzeugt hierbei also vorzugsweise ein optisches und/oder akustisches Warnsignal, das einen Rückschluss auf den Füllstand in dem Vorratsbehälter erlaubt. Die Erfassung der Füllstandsanzeige kann hierbei wahlweise auf elektrischem oder auf chemischem Wege erfolgen, jedoch ist die Erfindung auch mit anderen physikalischen Mechanismen zur Erfassung des Füllstandes in dem Vorratsbehälter realisierbar.

Als eine Variante ist vorgesehen, dass die leitfähige Elektrodenfläche mit einer möglichsten dünnen Gelschicht versehen ist, um den Übergangswiderstand zu Unterhaut(Impedanz) möglichst gering zu halten.

Dabei die Elektrodenfläche porös sein und die Erhaltung der Leitfähigkeit durch ein auf der körperabgewandten Seite der Elektrodenfläche angebrachtes Flüssigkeits- bzw. Gel-Reservoir erreicht werden. Dieses Reservoir liefert kontinuierlich leitfähige Flüssigkeit durch die poröse Elektrodenfläche in die körperzugewandte kontaktierende Gel-Schicht nach.

Darüber hinaus umfasst die Erfindung ein Verfahren zum Anlegen der vorstehend beschriebenen Elektrodenanordnung an ein Messobjekt, wie beispielsweise den Brustkorb eines bettlägerigen Patienten. Hierbei werden zunächst die Elektroden in Längsrichtung des gürtelförmigen Elektrodenträgers in vorgegebenen Positionen positioniert, woraufhin der Elektrodenträger mit den Elektroden dann an dem Messobjekt angebracht wird, wobei die Elektroden das Messobjekt elektrisch kontaktieren.

Die Positionierung der Elektroden in Längsrichtung des gürtelförmigen Elektrodenträgers vor dem Anbringen der erfindungsgemäßen Elektrodenanordnung an dem Messobjekt kann beispielsweise dadurch erfolgen, dass der gürtelförmige Elektrodenträger in Längs- bzw. Umfangsrichtung zwischen den Elektroden gedehnt wird, wobei der dadurch eingestellte und vorzugsweise gleichmäßige Abstand oder Winkelabstand zwischen den benachbarten Elektroden aufgrund der Plastizität des Materials des Elektrodenträgers anschließend beibehalten wird.

In einer vorteilhaften Variante der Erfindung wird zusätzlich eine Schablone verwendet, die an das Messobjekt angelegt wird. Beispielsweise kann die Schablone in Form eines Streifens um den Brustkorb eines Patienten herum gelegt werden, wobei die Überlänge der Schablone abgetrennt wird, so dass die Länge der Schablone gleich dem Umfang des Brustkorbs ist. Anschließend kann die Schablone dann mehrfach gefaltet werden, so dass entlang der Schablone äquidistante Falzstellen entstehen, die Markierungen zur Positionierung der einzelnen Elektroden bilden.

Darauf hin kann die Schablone dann von dem Brustkorb abgenommen und an den gürtelförmigen Elektrodenträger angelegt werden, wobei der Elektrodenträger so gedehnt wird, dass die Position der einzelnen Elektroden mit der Position der Falzstellen übereinstimmt.

Bei der vorstehend beschriebenen mehrteiligen Ausführung des gürtelförmigen Elektrodenträgers ist die Anbringung der erfindungsgemäßen Elektrodenanordnung insbesondere bei einem bettlägerigen Patienten besonders einfach. Hierzu wird zunächst mindestens ein Teil des gürtelförmigen Elektrodenträgers an dem freiliegenden Bereich des Brustkorbs des liegenden Patienten befestigt. Anschließend wird der Patient dann um seine Längsachse gedreht, woraufhin dann mindestens ein weiterer Teil des gürtelförmigen Elektrodenträgers an dem dann freiliegenden Bereich des Brustkorbs des Patienten befestigt wird. Anschließend können die einzelnen Teile des gürtelförmigen Elektrodenträgers dann jeweils paarweise elektrisch und/oder mechanisch miteinander verbunden werden. Vorzugsweise werden die einzelnen Teile des gürtelförmigen Elektrodenträgers jedoch unabhängig voneinander an dem Messobjekt befestigt und getrennt voneinander elektrisch angeschlossen.

Die erfindungsgemäße Elektrodenanordnung kann grundsätzlich eine beliebige Anzahl von Elektroden aufweisen. Vorzugsweise ist die Anzahl der Elektroden jedoch ein Vielfaches von zwei oder eine Zweierpotenz.

Andere vorteilhafte Weiterbildungen der Erfindung sind in den Unteransprüchen gekennzeichnet oder werden nachstehend zusammen mit der Beschreibung des bevorzugten Ausführungsbeispiels der Erfindung anhand der Figuren näher erläutert.
Es zeigen:
- Figur 1: eine erfindungsgemäße Elektrodenanordnung mit einem mehrteiligen gürtelförmigen Elektrodenträger sowie
- Figur 2a: eine vergrößerte Seitenschnittansicht eines Teils der erfindungsgemäßen Elektrodenanordnung aus Figur 1,
- Figur 2b: eine Seitenschnittansicht eines Teils der erfindungsgemäßen Elektrodenanordnung in normaler Größe,
- Figur 2c: eine Aufsicht eines Teils der erfindungsgemäßen Elektrodenanordnung,
- Figur 3: eine Querschnittsansicht eines Teils eines anderen Ausführungsbeispiels einer erfindungsgemäßen Elektrodenanordnung sowie
- Figur 4: eine Querschnittsansicht eines Ausschnitts aus Figur 3.

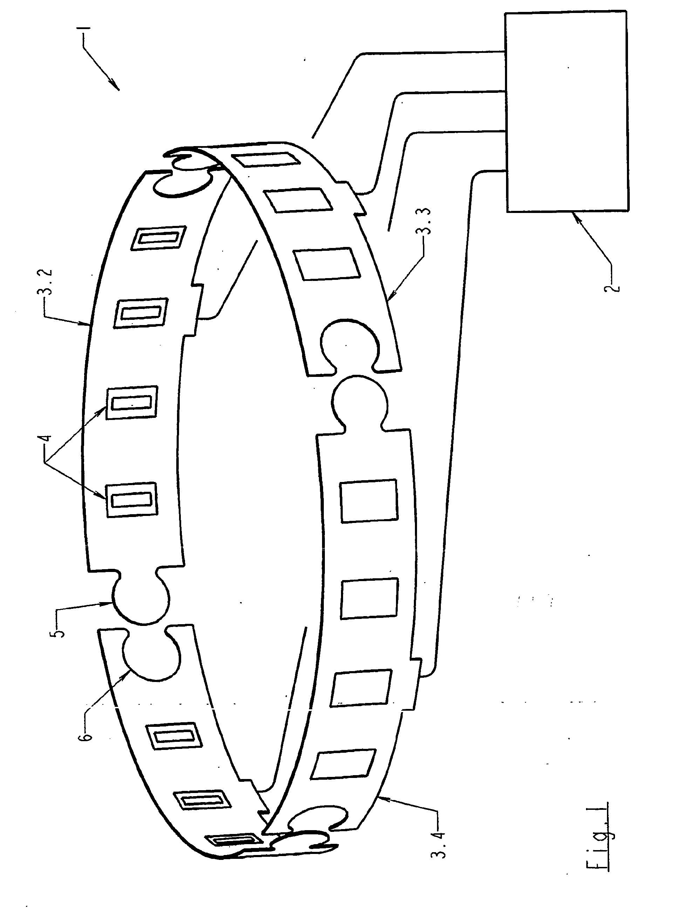

Die schematische Darstellung in Figur 1 zeigt eine erfindungsgemäße Elektrodenanordnung 1, die zur Durchführung einer Elektro-Impedanz-Tomographie die Messung mit einem herkömmlichen EIT-Gerät 2 verbunden werden kann.

Die Elektrodenanordnung 1 weist einen gürtelförmigen Elektrodenträger auf, der bei diesem Ausführungsbeispiel aus vier Teilen 3.1 - 3.4 besteht, wobei die einzelnen Teile 3.1 - 3.4 des Elektrodenträgers an ihrer dem Messobjekt zugewandten Seite jeweils vier Elektroden 4 tragen, die während der EIT-Messung den Brustkorb des Patienten elektrisch kontaktieren.

Die einzelnen Elektroden 4 sind jeweils rechteckig und rechtwinklig zur Längsachse bzw. zur Umfangsrichtung des gürtelförmigen Elektrodenträgers ausgerichtet.

Die einzelnen Teile 3.1 - 3.4 des gürtelförmigen Elektrodenträgers bestehen jeweils aus einem plastisch verformbaren Kunststoff, was eine Positionierung der Elektroden 4 in Umfangsrichtung ermöglicht, um während der Messung vorgegebene Elektrodenpositionen an dem Brustkorb des Patienten einhalten zu können.

Hierzu wird vor der Messung eine Schablone in Form eines Streifens um den Brustkorb des Patienten herum gelegt, wobei die Überlänge der Schablone abgeschnitten wird, so dass die Länge der Schablone mit dem Umfang des Brustkorbs übereinstimmt. Anschließend wird die Schablone dann entlang ihrer Längsrichtung viermal gefaltet, so dass äquidistante 15 Falzstellen entstehen, die jeweils eine Markierung zur Positionie-. rung der Elektroden 4 bilden. Anschließend wird die streifenförmige Schablone neben den Elektrodenträger gelegt, wobei die einzelnen Teile 3.1 - 3.4 des Elektrodenträgers so gedehnt werden, dass eine der Elektroden an einem Ende der Schablone liegt, während die Positionen der anderen Elektroden mit den Falzstellen der Schablone übereinstimmen.

Bei einer Untersuchung eines bettlägerigen Patienten durch das EIT-Verfahren wird dann zunächst das Teil 3.1 des gürtelförmigen Elektrodenträgers auf den freiliegenden Bereich des Brustkorbs des Patienten aufgelegt.

Anschließend wird der Patient dann so weit um seine Längsachse gedreht, dass das zweite Teil 3.2 des gürtelförmigen Elektrodenträgers an dem dann freiliegenden Bereich des Brustkorbs des Patienten angelegt werden kann.

Daraufhin werden die beiden Teile 3.1 und 3.2 elektrisch getrennt mit dem EIT-Gerät 2 verbunden. Hierzu dienen Anschlußleitungen mit jeweils einem farbkodierten Stecker, um Verwechslungen zu verhindern. Das Teil 3.1 kann beispielsweise eine Anschlußleitung mit einem blauen Stecker aufweisen, während der Stecker bei der Anschlußleitung für das Teil 3.2 rot ist. Bei dem Stecker für das Teil 3.3 kann der Stecker dann beispielsweise grün sein, während der Stecker für das Teil 3.4 gelb ist.

Die mechanische Verbindung zwischen den jeweils benachbarten Teilen 3.1-3.4 des gürtelförmigen Elektrodenträgers erfolgt dagegen durch Verbindungen, die aus zungenförmigen Steckern 5 und entsprechenden Aufnahmen 6 bestehen.

Dieser Vorgang wird dann anschließend für die Teile 3.3 und 3.4 des gürtelförmigen Elektrodenträgers wiederholt, bis schließlich sämtliche Teile 3.1 - 3.4 des gürtelförmigen Elektrodenträgers an den Brustkorb des Patienten angebracht sind, woraufhin die EIT-Tomographie beginnen kann.

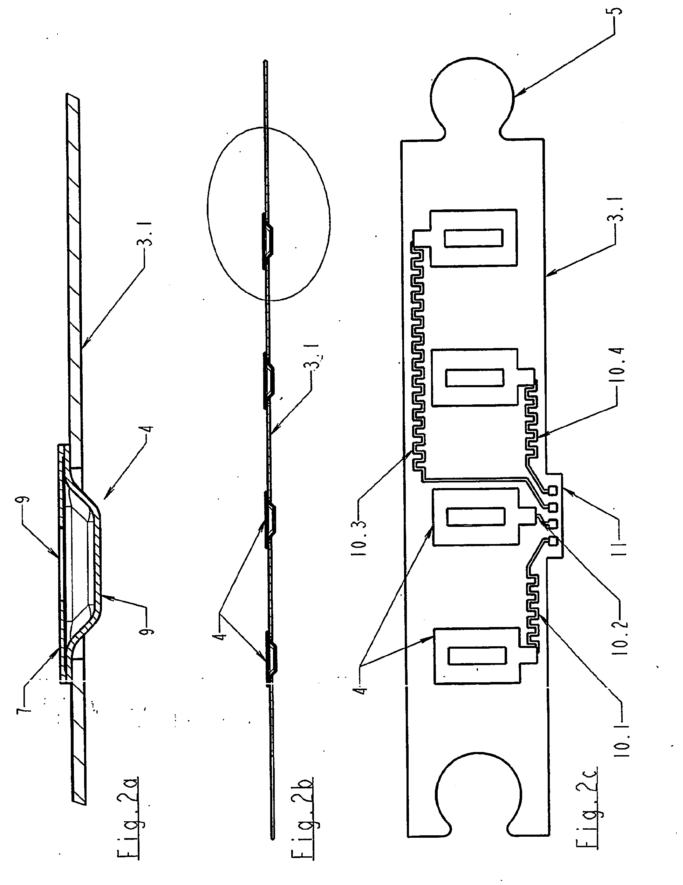

Die Figuren 2a und 2b zeigen Seitenschnittansichten eines der Teile 3.1-3.4 der erfindungsgemäßen Elektrodenanordnung, wobei aus Figur 2a auch der Aufbau der einzelnen Elektroden 4 ersichtlich ist.

So weist das Teil 3.1 des gürtelförmigen Elektrodenträgers im Bereich der Elektroden 4 jeweils einen rechteckigen Ausschnitt auf.

Auf der dem Messobjekt abgewandten Seite ist der Ausschnitt durch eine flüssigkeitsundurchlässige Schicht 7 abgedeckt, die an ihren Rändern mit dem Teil 3.1 verschweißt ist.

Auf der dem Messobjekt zugewandten Seite ist der Ausschnitt in dem Teil 3.1 des gürtelförmigen Elektrodenträgers dagegen durch eine teilweise permeable Membran 8 abgedeckt.

Die Schicht 7 und die Membran 8 schließen eine Kontaktflüssigkeit 9 ein, welche die Oberfläche des Messobjekts benetzt und dadurch den Ubergangswiderstand zwischen den Elektroden 4 und dem Messobjekt herabsetzt.

Weiterhin ist zu erwähnen, dass die Schicht 7 lichtdurchlässig ist, so dass die Untersuchungsperson den Flüssigkeitsstand in den Elektroden 4 einer einfachen Sichtprüfung unterziehen kann, um die Elektrodenanordnung rechtzeitig auswechseln zu können.

Aus Figur 2c ist ferner ersichtlich, das die einzelnen Elektroden 4 jeweils getrennte Anschlußleitungen 10.1-10.4 aufweisen, die an einer Verbindungsstelle 11 zusammengeführt sind, was eine Kontaktierung sämtlicher Elektroden 4 des Teils 3.1 durch eine einzige Steckverbindung ermöglicht.

Darüber hinaus sind die Anschlußleitungen 10.1, 10.3 und 10.4 im Bereich zwischen den einzelnen Elektroden 4 mäanderförmig geführt. Diese Führung der Anschlußleitungen 10.1, 10.3 und 10.4 bietet den Vorteil, dass die Anschlussleitungen 10.1, 10.3, 10.4 bei einer Dehnung des Teils 3.1 in Längs- bzw. Umfangsrichtung nur geringfügig deformiert werden, was einer Beschädigung entgegenwirkt.

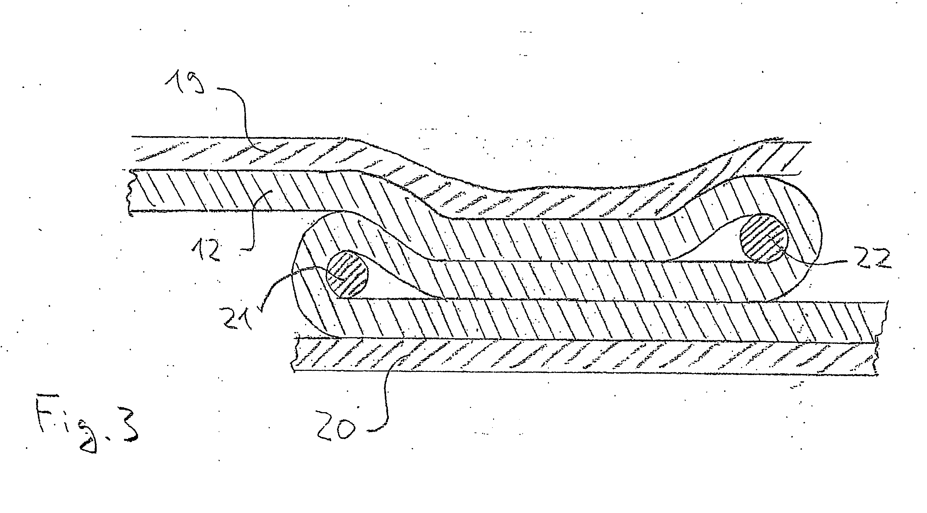

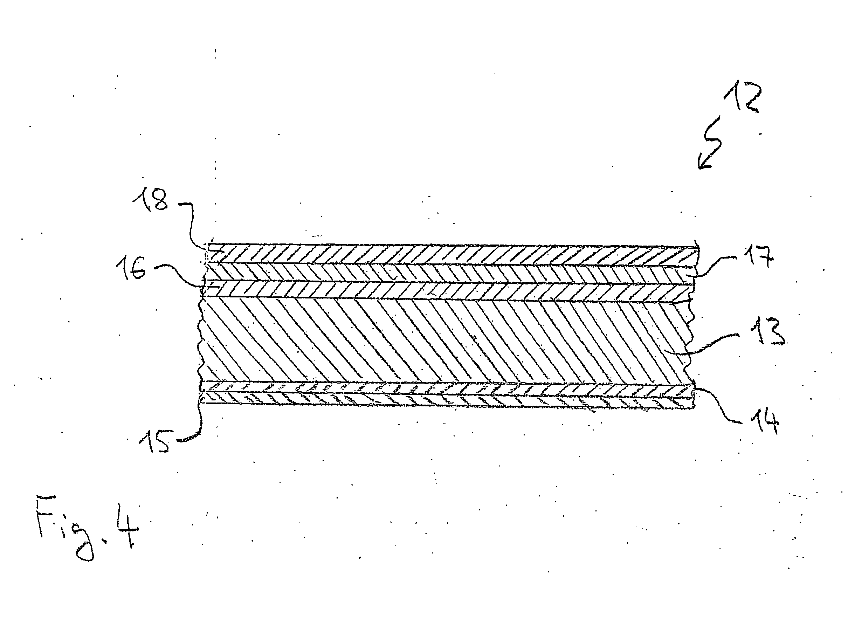

Schließlich zeigen die Figuren 3 und 4 eine Querschnittsansicht eines Elektrodenträgers 12, bei dem die Dehnbarkeit in Längsrichtung in besonderer Weise erreicht wird.

So weist der Elektrodenträger 12 als mechanisches Trägerelement eine aus Mylar bestehende Trägerfolie 13 auf, die an der dem Messobjekt zugewandten Seite mit einer Klebeschicht 14 bedeckt ist, um den Elektrodenträger 12 an dem Messobjekt festkleben zu können.

Bei der herstellerseitigen Auslieferung des Elektrodenträgers 12 ist die Klebeschicht 14 auf der dem Messobjekt zugewandten Seite mit einer abziehbaren Schutzschicht 15 abgedeckt, die vor der Anbringung an dem Messobjekt von der Untersuchungsperson abgezogen wird, um die Klebeschicht 14 freizulegen.

Auf der dem Messobjekt zugewandten Seite der Trägerfolie 13 sind in vorgegebenen Abständen Elektroden zur Kontaktierung des Messobjekts angeordnet, wobei die Elektroden in den Figuren 3 und 4 zur Vereinfachung nicht dargestellt sind.

Auf der dem Messobjekt abgewandten Seite der Trägerfolie 13 ist eine Leiterbahnschicht 16 aufgedruckt, um die Elektroden durch jeweils eine separate Anschlußleitung elektrisch zu kontaktieren.

Die Leiterbahnschicht 16 ist auf der dem Messobjekt abgewandten Seite wiederum mit einer elektrischen Isolierschicht 17 bedeckt, die Nebenschlüsse der Anschlußleitungen verhindert.

Schließlich ist auf die dem Messobjekt abgewandte Seite der Isolierschicht 17 eine Abschirmungsschicht 18 aufgebracht, welche die Anschlußleitungen der Elektroden elektrisch abschirmt und dadurch elektromagnetische Störungen unterdrückt und die Messgenauigkeit erhöht. Die Abschirmungsschicht 18 kann beispielsweise aus einer aufgedampften Metallschicht bestehen und wird vorzugsweise mit den Anschlußleitungen für die Elektroden zu einem elektrischen Anschluß geführt und dort auf Massepotential gelegt, um eine gute Abschirmwirkung zu erreichen.

Die Abschirmung kann sowohl ganzflächig als auch je Einzelelektrode ausgebildet sein und auf der gegenüberliegenden Seite des Elektrodenträgers identisch bzw. geringfügig breiter geführt werden. Die Abschirmung kann dabei sowohl gemeinsam geerdet, als auch je Einzelelektrode aktiv mit dem jeweiligen Einzelsignal zur Gleichtaktunterdrückung getrieben sein.

Die elektrische Kontaktierung von Elektrodenbahnen und Abschirmungsbahnen am Stecker erfolgt vorzugsweise durch einen doppelseitigen Steckverbinder. Dieser doppelseitige Stecker kann durch eine Verstärkung am Elektrodenträger angesteckt und z.B. durch eine Druckverbindung gesichert kontaktiert werden.

Zwischen den in Längsrichtung benachbarten Elektroden ist der Elektrodenträger 12 jeweils in mehreren Lagen übereinander gefaltet, wie aus Figur 3 ersichtlich ist. Dies ermöglicht eine Dehnung des Elektrodenträgers 12 in Längsrichtung, indem der Elektrodenträger 12 von der Untersuchungsperson in Längsrichtung auseinander gezogen wird, wobei sich der Elektrodenträger 12 entfaltet.

Zur Verhinderung einer unerwünschten selbständigen Entfaltung des Elektrodenträgers 12 sind die übereinander liegenden Lagen des Elektrodenträgers 12 beidseitig mit einem Klebeband 19, 20 abgedeckt, das die einzelnen übereinander gefalteten Lagen in der jeweiligen Position fixiert, sofern die Untersuchungsperson den Elektrodenträger 12 nicht in Längsrichtung auseinander zieht. Die Klebebänder 19, 20 stellen also sicher, dass sich der Elektrodenträger 12 bei Transport und Lagerung sowie während der eigentlichen Untersuchung nicht selbständig entfaltet, was zu einer ungewollten und undefinierten Änderung der Elektrodenpositionen führen würde.

Schließlich befindet sich zwischen den einzelnen übereinander gefalteten Lagen des Elektrodenträgers 12 in den Faltkanten jeweils ein Schutzelement 21, 22, um eine Knickbildung zu verhindern. Dies ist vorteilhaft, da eine Knickbildung die Entfaltung des Elektrodenträgers 12 und damit die freie Positionierung der Elektroden behindern würde. Die Schutzelement 21, 22 weisen einen kreisförmigen Querschnitt auf und haben einen Außendurchmesser, der geringfügig größer ist als die Dicke des Elektrodenträgers 12.

Die Erfindung ist nicht auf das vorstehend beschriebene Ausführungsbeispiel beschränkt. Vielmehr ist eine Vielzahl von Varianten und Abwandlungen möglich, die ebenfalls von dem Erfindungsgedanken Gebrauch machen und deshalb in den Schutzbereich fallen.

## Patentansprüche

1. Elektrodenanordnung (1), insbesondere für die Elektro-Impedanz-Tomographie, mit
mehreren Elektroden (4) zur elektrischen Kontaktierung eines Messobjekts,
einem gürtelförmigen Elektrodenträger (3.1-3.4, 12) zur Umfassung des Messobjekts,
wobei die Elektroden (4) an dem gürtelförmigen Elektrodenträger (3.1-3.4; 12) angebracht sind,
wobei die Elektroden (4) an dem gürtelförmigen Elektrodenträger (3.1-3.4, 12) in Längsrichtung des gürtelförmigen Elektrodenträgers (3.1-3.4, 12) positionierbar sind,
**dadurch gekennzeichnet, dass**
der gürtelförmige Elektrodenträger (3.1-3.4, 12) aus mehreren Teilen besteht, die im montierten Zustand an dem Messobjekt in Längsrichtung des Elektrodenträgers (3.1-3.4, 12) aneinander angrenzen und jeweils paarweise mechanisch miteinander verbunden werden können.

2. Elektrodenanordnung (1) nach Anspruch 1, **dadurch gekennzeichnet, dass** die Elektroden (4) an dem gürtelförmigen Elektrodenträger (3.1-3.4, 12) in Längsrichtung des gürtelförmigen Elektrodenträgers (3.1-3.4, 12) frei und bleibend positionierbar sind.

3. Elektrodenanordnung (1) nach Anspruch 1 und/oder Anspruch 2, **dadurch gekennzeichnet, dass** der gürtelförmige Elektrodenträger (3.1-3.4, 12) zur Ermöglichung der Positionierbarkeit der Elektroden (4) mindestens teilweise aus einem plastisch verformbaren Material besteht.

4. Elektrodenanordnung nach mindestens einem der vorhergehenden Ansprüche, **dadurch gekennzeichnet, dass** der Elektrodenträger (12) zwischen zwei benachbarten Elektroden in mehreren Lagen übereinander gefaltet ist.

5. Elektrodenanordnung nach Anspruch 4, **dadurch gekennzeichnet, dass** zwischen den einzelnen Lagen des Elektrodenträgers (12) in mindestens einer Faltkante des Elektrodenträgers (12) mindestens ein Schutzelement (21, 22) zur Verhinderung einer Knickbildung angeordnet sind.

6. Elektrodenanordnung nach Anspruch 4 und/oder Anspruch 5, **dadurch gekennzeichnet, dass** die übereinander gefalteten Lagen des Elektrodenträgers (12) zur Verhinderung einer selbständigen Entfaltung durch mindestens ein Klebeelement (19, 20) fixiert sind.

7. Elektrodenanordnung nach Anspruch 6, **dadurch gekennzeichnet, dass** die übereinander gefalteten Lagen des Elektrodenträgers (12) zur Verhinderung einer selbständigen Entfaltung beidseitig mit einem Klebeband (19, 20) bedeckt sind.

8. Elektrodenanordnung (1) nach mindestens einem der vorhergehenden Ansprüche, **dadurch gekennzeichnet, dass** sich der Elektrodenträger (3.1-3.4, 12) bei einer Dehnung in Längsrichtung über seine Länge im wesentlichen gleichmäßig dehnt.

9. Elektrodenanordnung (1) nach mindestens einem der vorhergehenden Ansprüche, **dadurch gekennzeichnet, dass** das plastisch verformbare Material ein Kunststoff oder ein Gewebe mit Kunststoffbeschichtung ist.

10. Elektrodenanordnung (1) nach mindestens einem der vorhergehenden Ansprüche, **dadurch gekennzeichnet, dass** zur elektrischen Kontaktierung der Elektroden (4) Anschlußleitungen (10.1-10.4) vorgesehen sind, wobei die Anschlußleitungen (10.1-10.4) an dem gürtelförmigen Elektrodenträger (3.1-3.4) angebracht und in Längsrichtung des gürtelförmigen Elektrodenträgers (3.1-3.4) dehnbar sind.

11. Elektrodenanordnung (1) nach Anspruch 10, **dadurch gekennzeichnet, dass** die Anschlußleitungen (10.1-10.4) an dem gürtelförmigen Elektrodenträger (3.1-3.4) mäanderförmig verlaufen.

12. Elektrodenanordnung (1) nach Anspruch 10 und/oder Anspruch 11, **dadurch gekennzeichnet, dass** die Anschlußleitungen (10.1-10.4) aus einem elektrisch leitfähigen und dehnbaren Material bestehen.

13. Elektrodenanordnung nach mindestens einem der vorhergehenden Ansprüche, **dadurch gekennzeichnet, dass** der Elektrodenträger eine Folie ist, wobei die Elektroden und/oder die Anschlußleitungen auf die Folie aufgebracht sind.

14. Elektrodenanordnung (1) nach mindestens einem der vorhergehenden Ansprüche, **dadurch gekennzeichnet, dass** der Elektrodenträger auf der dem Messobjekt zugewandten Seite und/oder auf der dem Messobjekt abgewandten Seite mindestens teilweise mit einer elektrischen Isolierschicht bedeckt ist.

15. Elektrodenanordnung (1).nach mindestens einem der vorhergehenden Ansprüche, **dadurch gekennzeichnet, dass** der Elektrodenträger auf der dem Messobjekt zugewandten Seite und/oder auf der dem Messobjekt abgewandten Seite mindestens teilweise mit einer elektrischen Abschirmung bedeckt ist.

16. Elektrodenanordnung (1) nach Anspruch 15, **dadurch gekennzeichnet, dass** die Abschirmung ganzflächig ausgebildet oder auf den Bereich der Elektroden beschränkt ist.

17. Elektrodenanordnung (1) nach Anspruch 15 und/oder Anspruch 16, **dadurch gekennzeichnet, dass** die Abschirmung geerdet oder mit dem jeweiligen Einzelsignal zur Gleichtaktunterdrückung beaufschlagt ist.

18. Elektrodenanordnung (1) nach mindestens einem der vorhergehenden Ansprüche, **dadurch gekennzeichnet, dass** zur mechanischen Verbindung aneinander angrenzender Teile des gürtelförmigen Elektrodenträgers (3.1-3.4) eine Fügeverbindung oder eine Crimpverbindung vorgesehen ist.

19. Elektrodenanordnung (1) nach mindestens einem der vorhergehenden Ansprüche, **dadurch gekennzeichnet, dass** zur elektrischen Kontaktierung des Messobjekts eine Kontaktflüssigkeit vorgesehen ist, die in einem Vorratsbehälter (9) bevorratet wird, wobei der Vorratsbehälter (9) an dem gürtelförmigen Elektrodenträger (3.1-3.4) angebracht ist.

20. Elektrodenanordnung (1) nach Anspruch 19, **dadurch gekennzeichnet, dass** der Vorratsbehälter (9) in die Elektroden (4) integriert ist.

21. Elektrodenanordnung (1) nach Anspruch 19 und/oder Anspruch 20, **gekennzeichnet durch** eine optische und/oder akustische Füllstandsanzeige für den Vorratsbehälter.

22. Elektrodenanordnung (1) nach Anspruch 21, **gekennzeichnet durch**, dass die Füllstandsanzeige den Füllstand elektrisch oder chemisch erfasst.

23. Elektrodenanordnung nach mindestens einem der vorhergehenden Ansprüche, **dadurch gekennzeichnet, dass** die Elektroden mindestens teilweise mit einer Gel-Schicht beschichtet sind.

24. Verfahren zum Anlegen einer Elektrodenanordnung (1) nach mindestens einem der vorhergehenden Ansprüche an ein Messobjekt, mit den folgenden Schritten:
- Positionierung der Elektroden (4) in Längsrichtung des gürtelförmigen Elektrodenträgers (3.1-3.4) in vorgegebenen Positionen
- Anbringung des Elektrodenträgers an dem Messobjekt, wobei die Elektroden (4) das Messobjekt kontaktieren.

25. Verfahren nach Anspruch 24, **dadurch gekennzeichnet, dass** der gürtelförmige Elektrodenträger (3.1-,3.4) zur Positionierung der Elektroden (4) jeweils in Längsrichtung des Elektrodenträgers (3.1-3.4) zwischen den Elektroden (4) gedehnt wird.

26. Verfahren nach Anspruch 24 und/oder Anspruch 25, **gekennzeichnet durch** folgende Schritte:
- Umschlingung des Messobjekts mit einer Schablone
- Kürzen der Schablone auf den Umfang des Messobjekts
- Erzeugung von äquidistanten Markierungen an der Schablone
- Anlegen der Schablone an den gürtelförmigen Elektrodenträger (3.1-3.4),
- Äquidistante Positionierung der Elektroden (4) mittels Dehnung an dem gürtelförmigem Elektrodenträger (3.1-3.4) entsprechend den Markierungen an der Schablone.

27. Verfahren nach Anspruch 26, **dadurch gekennzeichnet, dass** die Schablone vor dem Anlegen an den Elektrodenträger von dem Messobjekt abgenommen wird.

28. Verfahren nach mindestens einem der Ansprüche 24 bis 26, **dadurch gekennzeichnet, dass** das Messobjekt ein Körperteil eines Menschen ist, wobei folgende Schritte ausgeführt werden:
- Anbringung mindestens eines Teils des gürtelförmigen Elektrodenträgers (3.1-3.4) auf dem freiliegenden Teil des Körperteils
- Drehung des Körperteils
- Anbringung mindestens eines weiteren Teils des gürtelförmigen Elektrodenträgers (3.1-3.4) an dem dann freiliegenden Teil des Körperteils
- Verbindung der Teile des gürtelförmigen Elektrodenträgers (3.1-3.4) jeweils paarweise miteinander.

29. Verfahren nach Anspruch 28, **dadurch gekennzeichnet, dass** das Körperteil ein Thorax, ein Hals oder ein Kopf ist.

## Claims

1. Electrode arrangement (1), in particular for electric impedance tomography, with a plurality of electrodes (4) for establishing electrical contact with an object to be measured, a belt-like electrode support (3.1 - 3.4, 12) for encircling the object to be measured, wherein the electrodes (4) are mounted on the belt-like electrode support (3.1 - 3.4, 12), wherein the electrodes (4) can be positioned on the belt-like electrode support (3.1 - 3.4, 12) in the longitudinal direction of the belt-like electrode support (3.1 - 3.4, 12), **characterised in that** the belt-like electrode support (3.1 - 3.4, 12) comprises a plurality of parts which adjoin one another in the longitudinal direction of the electrode support (3.1 - 3.4, 12) and can be connected to one another mechanically in pairs.

2. Electrode arrangement (1) as claimed in Claim 1, **characterised in that** the electrodes (4) can be positioned freely and permanently on the belt-like electrode support (3.1 - 3.4, 12) in the longitudinal direction of the belt-like electrode support (3.1 - 3.4, 12).

3. Electrode arrangement (1) as claimed in Claim 1 and/or Claim 2, **characterised in that** the belt-like electrode support (3.1 - 3.4, 12) is made at least in part from a plastically deformable material in order to facilitate the positioning of the electrodes (4).

4. Electrode arrangement as claimed in at least one of the preceding claims, **characterised in that** between two adjacent electrodes the electrode support (12) is folded in several layers one over the other.

5. Electrode arrangement as claimed in Claim 4, **characterised in that** between the individual layers of the electrode support (12) at least one protective element (21, 22) is disposed in at least one folded edge of the electrode support (12) in order to prevent kinking.

6. Electrode arrangement as claimed in Claim 4 and/or Claim 5, **characterised in that** the layers of the electrode support (12) which are folded one over the other are fixed by at least one adhesive element (19, 20) in order to prevent autonomous unfolding.

7. Electrode arrangement as claimed in Claim 6, **characterised in that** the layers of the electrode support (12) which are folded one over the other are covered on both sides with an adhesive strip (19, 20) in order to prevent autonomous unfolding.

8. Electrode arrangement (1) as claimed in at least one of the preceding claims, **characterised in that** in the event of stretching in the longitudinal direction the electrode support (3.1 - 3.4, 12) stretches substantially uniformly over its length.

9. Electrode arrangement (1) as claimed in at least one of the preceding claims, **characterised in that** the plastically deformable material is a plastics material or a fabric with a coating of plastics material.

10. Electrode arrangement (1) as claimed in at least one of the preceding claims, **characterised in that** connecting leads (10.1 - 10.4) are provided for establishing electrical connection with the electrodes (4), wherein the connecting leads (10.1 - 10.4) are attached to the belt-like electrode support (3.1 - 3.4) and can stretch in the longitudinal direction of the belt-like electrode support (3.1 - 3.4).

11. Electrode arrangement (1) as claimed in Claim 10, **characterised in that** the connecting leads (10.1 - 10.4) extend in meandering form on the belt-like electrode support (3.1-3.4).

12. Electrode arrangement (1) as claimed in Claim 10 and/or Claim 11, **characterised in that** the connecting leads (10.1 - 10.4) are made from an electrically conductive material which can stretch.

13. Electrode arrangement as claimed in at least one of the preceding claims, **characterised in that** the electrode support is a film, the electrodes and/or the connecting leads being attached to the film.

14. Electrode arrangement (1) as claimed in at least one of the preceding claims, **characterised in that** the electrode support is covered at least partially by an electrically insulating layer on the side facing the object to be measured and/or on the side facing away from the object to be measured.

15. Electrode arrangement (1) as claimed in at least one of the preceding claims, **characterised in that** the electrode support is covered at least partially by an electric shielding on the side facing the object to be measured and/or on the side facing away from the object to be measured.

16. Electrode arrangement (1) as claimed in Claim 15, **characterised in that** the shielding is constructed over the entire surface or is restricted to the region of the electrodes.

17. Electrode arrangement (1) as claimed in Claim 15 and/or Claim 16, **characterised in that** the shielding is earthed or is supplied with the respective individual signal for common-mode rejection.

18. Electrode arrangement (1) as claimed in at least one of the preceding claims, **characterised in that** a jointed connected or a crimped connection is provided for the mechanical connection of adjoining parts of the belt-like electrode support (3.1 - 3.4).

19. Electrode arrangement (1) as claimed in at least one of the preceding claims, **characterised in that** for establishing electrical contact with the object to be measured a contact fluid is provided which is stored in a storage container (9), the storage container (9) being attached to the belt-like electrode support (3.1 - 3.4).

20. Electrode arrangement (1) as claimed in Claim 19, **characterised in that** the storage container (9) is integrated into the electrodes (4).

21. Electrode arrangement (1) as claimed in Claim 19 and/or Claim 20, **characterised by** an optical and/or acoustic filling level indicator for the storage container.

22. Electrode arrangement (1) as claimed in Claim 21, **characterised in that** the filling level indicator detects the filling level electrically or chemically.

23. Electrode arrangement as claimed in at least one of the preceding claims, **characterised in that** the electrodes are coated at least partially with a gel layer.

24. Method of applying an electrode arrangement (1) as claimed in at least one of the preceding claims to an object to be measured, with the following steps:
- positioning of the electrodes (4) in the longitudinal direction of the belt-like electrode support (3.1 - 3.4) in predetermined positions,
- attachment of the electrode to the object to be measured, whereby the electrodes (4) establish contact with the object to be measured.

25. Method as claimed in Claim 24, **characterised in that** for positioning of the electrodes (4) the.belt-like electrode support (3.1 - 3.4) is stretched between the electrodes (4) in each case in the longitudinal direction of the electrode support (3.1 - 3.4).

26. Method as claimed in Claim 24 and/or Claim 25, **characterised by** the following steps:
- surrounding the object to be measured with a template,
- shortening the template to the periphery of the object to be measured,
- producing equidistant markings on the template,
- placing the template on the belt-like electrode support (3.1 - 3.4),
- equidistant positioning of the electrodes (4) by means of stretching on the belt-like electrode support (3.1 - 3.4) according to the markings on the template.

27. Method as claimed in Claim 26, **characterised in that** the template is removed from the object to be measured before the latter is placed on the electrode support.

28. Method as claimed in at least one of Claims 24 to 26, **characterised in that** the object to be measured is a body part of a human being, wherein the following steps are carried out:
- attachment of at least a part of the belt-like electrode support (3.1 - 3.4) to the exposed part of the body part,
- turning of the body part,
- attachment of at least one further part of the belt-like electrode support (3.1 - 3.4) to the part of the body part which is then exposed,
- connection of the parts of the belt-like electrode support (3.1 - 3.4) to one another in each case in pairs.

29. Method as claimed in Claim 28, **characterised in that** the body part is a thorax, a neck or a head.

## Revendications

1. Agencement d'électrodes (1), en particulier pour la tomographie d'impédance électrique, comprenant
plusieurs électrodes (4) pour la connexion électrique d'un objet de mesure,
un support d'électrodes en forme de ceinture (3.1 à 3.4, 12) pour entourer l'objet de mesure,
les électrodes (4) étant appliquées sur le support d'électrodes en forme de ceinture (3.1 à 3.4),
les électrodes (4) sur le support d'électrodes en forme de ceinture (3.1 à 3.4) pouvant être positionnées dans la direction longitudinale du support d'électrodes en forme de ceinture (3.1 à 3.4),
**caractérisé en ce que**
le support d'électrodes en forme de ceinture (3.1 à 3.4, 12) est composé de plusieurs parties qui sont adjacentes les unes aux autres dans la direction longitudinale du support d'électrodes (3.1 à 3.4, 12) lorsqu'elles sont montées sur l'objet de mesure et peuvent être reliées entre elles par paires mécaniquement.

2. Agencement d'électrodes (1) selon la revendication 1, **caractérisé en ce que** les électrodes (4) sont positionnables librement de manière durable sur le support d'électrodes (3.1 à 3.4, 12) dans la direction longitudinale du support d'électrodes en forme de ceinture (3.1 à 3.4, 12).

3. Agencement d'électrodes (1) selon la revendication 1 et/ou la revendication 2, **caractérisé en ce que** le support d'électrodes en forme de ceinture (3.1 à 3.4, 12) est réalisé au moins en partie dans une matière déformable plastiquement.

4. Agencement d'électrodes selon au moins l'une quelconque des revendications précédentes, **caractérisé en ce que** le support d'électrodes (12) est plié en plusieurs couches superposées entre deux électrodes.

5. Agencement d'électrodes selon la revendication 4, **caractérisé en ce qu'**au moins un élément de protection (21, 22) est disposé entre les différentes couches du support d'électrodes (12) dans au moins une pliure du support d'électrodes (12) pour éviter une formation de coude.

6. Agencement d'électrodes selon la revendication 4 et/ou la revendication 5, **caractérisé en ce que** les couches pliées l'une au-dessus de l'autre du support d'électrodes (12) sont fixées par au moins un élément adhésif (19, 20) pour empêcher un déploiement automatique.

7. Agencement d'électrodes selon la revendication 6, **caractérisé en ce que** les couches pliées les unes sur les autres du support d'électrodes (12) sont recouvertes des deux côtés par une bande adhésive (19, 20) pour empêcher une déploiement automatique.

8. Agencement d'électrodes (1) selon au moins l'une quelconque des revendications précédentes, **caractérisé en ce que** le support d'électrodes (3.1 à 3.4, 12) s'étend essentiellement uniformément sur sa longueur lors d'une extension dans la direction longitudinale.

9. Agencement d'électrodes (1) selon au moins l'une quelconque des revendications précédentes, **caractérisé en ce que** la matière déformable plastiquement est une matière plastique ou un textile avec une enduction de matière plastique.

10. Agencement d'électrodes (1) selon au moins l'une quelconque des revendications précédentes, **caractérisé en ce que**, pour la connexion électrique des électrodes (4), il est prévu des lignes de raccordement (10.1 à 10.4) qui sont posées sur le support d'électrodes en forme de ceinture (3.1 à 3.4) et sont extensibles dans la direction longitudinale du support d'électrodes en forme de ceinture (4.1 à 3.4)

11. Agencement d'électrodes (1) selon au moins l'une quelconque des revendications précédentes, **caractérisé en ce que** les lignes de raccordement (10.1 à 10.4) s'étendent en méandres sur le support d'électrodes (3.1 à 3.4) en forme de ceinture.

12. Agencement d'électrodes (1) selon au moins l'une quelconque des revendications précédentes, **caractérisé en ce que** les lignes de raccordement (10.1 à 10.4) sont réalisées dans une matière conductrice de l'électricité et extensible.

13. Agencement d'électrodes (1) selon au moins l'une quelconque des revendications précédentes, **caractérisé en ce que** le support d'électrodes est un film, les électrodes et/ou les lignes de raccordement étant appliquées sur le film.

14. Agencement d'électrodes (1) selon au moins l'une quelconque des revendications précédentes, **caractérisé en ce que** le support d'électrodes, du côté tourné vers l'objet de mesure et/ou du côté opposé à l'objet de mesure, est recouvert au moins en partie par une couche isolant de l'électricité.

15. Agencement d'électrodes (1) selon au moins l'une quelconque des revendications précédentes, **caractérisé en ce que** le support d'électrodes, du côté tourné vers l'objet de mesure et/ou du côté opposé à l'objet de mesure, est recouvert au moins en partie par un blindage contre l'électricité.

16. Agencement d'électrodes (1) selon la revendication 15, **caractérisé en ce que** le blindage est réalisé sur toute la surface ou est limité à la zone des électrodes.

17. Agencement d'électrodes (1) selon la revendication 15 et/ou la revendication 16, **caractérisé en ce que** le blindage est mis à la terre ou est poussé avec le signal individuel vers la réjection en mode commun.

18. Agencement d'électrodes (1) selon au moins l'une quelconque des revendications précédentes, **caractérisé en ce que**, pour la liaison mécanique de parties du support d'électrodes en forme de ceinture (3.1 à 3.4) adjacentes les unes aux autres, il est prévu une jointure ou une sertissure.

19. Agencement d'électrodes (1) selon au moins l'une quelconque des revendications précédentes, **caractérisé en ce que**, pour la connexion électrique de l'objet de mesure, il est prévu un liquide de contact qui est stocké dans un réservoir de mesure (9), lequel est disposé sur le support d'électrodes en forme de ceinture (3.1 à 3.4).

20. Agencement d'électrodes (1) selon la revendication 19, **caractérisé en ce que** le réservoir de stockage (9) est intégré dans les électrodes (4).

21. Agencement d'électrodes (1) selon la revendication 19 et/ou la revendication 20, **caractérisé par** une indication de niveau optique et/ou acoustique pour le réservoir de stockage.

22. Agencement d'électrodes (1) selon la revendication 21, **caractérisé en ce que** l'indication de niveau détecte le niveau électriquement ou chimiquement.

23. Agencement d'électrodes (1) selon au moins l'une quelconque des revendications précédentes, **caractérisé en ce que** les électrodes sont revêtues au moins en partie d'une couche de gel.

24. Procédé pour appliquer un agencement d'électrodes (1) selon au moins l'une quelconque des revendications précédentes sur un objet de mesure, comprenant les étapes suivantes :
- positionnement des électrodes (1) dans la direction longitudinale du support d'électrodes (3.1 à 3.4) dans des positions prédéterminées
- application du support d'électrodes sur l'objet de mesure les électrodes (4) établissant le contact avec l'objet de mesure.

25. Procédé selon la revendication 24, **caractérisé en ce que**, pour le positionnement des électrodes (4), le support d'électrodes en forme de ceinture (3.1 à 3 .4) est étiré entre les électrodes (4) dans la direction longitudinale du support d'électrodes (3.1 à 3.4).

26. Procédé selon la revendication 24 et/ou la revendication 25, **caractérisé par** les étapes suivantes:
- enlacement de l'objet de mesure avec un gabarit
- raccourcissement du gabarit à la circonférence de l'objet de mesure
- production de marques équidistantes sur le gabarit
- pose du gabarit sur le support d'électrodes en forme de ceinture (3.1 à 3.4)
- positionnement équidistant des électrodes (4) par extension sur le support d'électrodes en forme de ceinture (3.1 à 3.4) de manière correspondant aux marques du gabarit.

27. Procédé selon la revendication 26, **caractérisé en ce que** le gabarit est enlevé de l'objet de mesure avant la pose sur le support d'électrodes.

28. Procédé selon l'au moins l'une des revendications 24 à 26, **caractérisé en ce que** l'objet de mesure est une partie du corps d'un être humain, les étapes suivantes étant exécutées :
- pose d'au moins une partie du support d'électrodes en forme de ceinture (3.1 à 3.4) sur la région dégagée de la partie du corps
- rotation de la partie du corps
- pose d'au moins une autre partie de l'électrode en forme de ceinture (3.1 à 3.4) sur la région ensuite dégagée de la partie du corps
- liaison des parties du support d'électrodes en forme de ceinture (3.1 à 3.4) par paires entre elles.

29. Procédé selon la revendication 28, **caractérisé en ce que** la partie du corps est un thorax, un cou ou une tête.
